# EUROPEAN PATENT APPLICATION

(11) **EP 4 268 748 A2**
(43) Date of publication of application: **01.11.2023**
(21) Application number: 23170297.8
(22) Date of filing: 27.04.2023
(51) Int. Cl.: A61B 18/14, A61B 18/00, A61B 5/00

(54) **IRRIGATION HUB FOR AN ABLATION CATHETER**

(30) Priority: 28.04.2022 US 202263336023 P; 28.04.2022 US 202263336094 P; 29.12.2022 US 202263477819 P; 29.03.2023 US 202318192361
(71) Applicant: BIOSENSE WEBSTER (ISRAEL) LTD., 2066717 Yokneam (IL)
(72) Inventor: BEECKLER, Christopher Thomas, Irvine, 92618 (US); KEYES, Joseph Thomas, Irvine, 92618 (US); OKARSKI, Kevin Mark, Irvine, 92618 (US)
(74) Representative: Carpmaels & Ransford LLP

(57) **Abstract**

The disclosed technology includes an irrigation hub for an ablation catheter comprising a cylindrical member extending along a longitudinal axis. The cylindrical member can comprise a proximal end having a first outer diameter and a recess extending inwardly along the longitudinal axis forming an interior portion, a distal end having a second outer diameter, the second outer diameter being less than the first outer diameter, and an irrigation inlet chamber disposed proximate the interior portion and configured to receive fluid from an irrigation supply. The cylindrical member can further comprise a plurality of irrigation openings disposed generally transverse to the longitudinal axis from a distal portion of the irrigation inlet chamber and a flow diverter extending into the distal portion of the irrigation inlet chamber to block fluid flow and redirect fluid flow out of the plurality of irrigation openings in a direction generally transverse relative to the longitudinal axis.

## Description

### REFERENCE TO RELATED APPLICATIONS

This application claims the benefit of priority under 35 U.S.C. § 119 to prior filed U.S. Provisional Patent Application No. 63/336,023 (Attorney Docket No.: BIO6675USPSP1) filed on April 28, 2022, prior filed U.S. Provisional Patent Application No. 63/336,094 (Attorney Docket No.: BIO6693USPSP1) filed on April 28, 2023, and prior filed U.S. Provisional Patent Application No. 63/477,819 (BIO6794USPSP1) filed on December 29, 2022, the entire contents of each of which is hereby incorporated by reference as if set forth in full herein.

### FIELD

The present invention relates generally to medical devices, and in particular medical probes with irrigation, and further relates to, but not exclusively, medical probes configured to provide irrigation to electrodes.

### BACKGROUND

Cardiac arrhythmias, such as atrial fibrillation (AF), occur when regions of cardiac tissue abnormally conduct electrical signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. By selectively ablating cardiac tissue by application of energy via a catheter, it is sometimes possible to cease or modify the propagation of unwanted electrical signals from one portion of the heart to another.

Many current ablation approaches in the art utilize radiofrequency (RF) electrical energy to heat tissue. RF ablation can have certain risks related to thermal heating which can lead to tissue charring, burning, steam pop, phrenic nerve palsy, pulmonary vein stenosis, and esophageal fistula.

Cryoablation is an alternative approach to RF ablation that generally reduces thermal risks associated with RF ablation. Maneuvering cryoablation devices and selectively applying cryoablation, however, is generally more challenging compared to RF ablation; therefore, cryoablation is not viable in certain anatomical geometries which may be reached by electrical ablation devices.

Some ablation approaches use irreversible electroporation (IRE) to ablate cardiac tissue using nonthermal ablation methods. IRE delivers short pulses of high voltage to tissues and generates an unrecoverable permeabilization of cell membranes. Delivery of IRE energy to tissues using multi-electrode probes was previously proposed in the patent literature. Examples of systems and devices configured for IRE ablation are disclosed in U.S. Patent Pub. No. 2021/0169550A1, 2021/0169567A1, 2021/0169568A1, 2021/0161592A1, 2021/0196372A1, 2021/0177503A1, and 2021/0186604A1, each of which are incorporated herein by reference and attached in the Appendix hereto.

Ablation of tissue can cause localized temperature increases near the electrodes. Therefore, many existing ablation catheters include irrigation elements that are configured deliver irrigation to areas proximate the electrodes. For example, some existing ablation catheters are configured to deliver saline to areas proximate the electrodes. Unfortunately, many existing irrigation elements are designed with sharp turns or otherwise inefficient designs that can reduce the effectiveness of the cooling provided by the irrigation elements. Accordingly, there is a need in the art for irrigation elements that increase the effectiveness of the cooling provided by the irrigation elements.

### SUMMARY

There is provided, in accordance with an example of the present invention, an irrigation hub for an ablation catheter. The irrigation hub can comprise a cylindrical member extending along a longitudinal axis. The cylindrical member can comprise a proximal end having a first outer diameter and a recess extending inwardly along the longitudinal axis forming an interior portion, a distal end having a second outer diameter, the second outer diameter being less than the first outer diameter, and an irrigation inlet chamber disposed proximate the interior portion and configured to receive fluid from an irrigation supply. The cylindrical member can further comprise a plurality of irrigation openings disposed generally transverse to the longitudinal axis from a distal portion of the irrigation inlet chamber and a flow diverter extending into the distal portion of the irrigation inlet chamber to block fluid flow and redirect fluid flow out of the plurality of irrigation openings in a direction generally transverse relative to the longitudinal axis.

The disclosed technology can include a medical probe comprising a tubular shaft extending along a longitudinal axis of the medical probe, a plurality of spines configured to bow radially outward from the longitudinal axis, and a plurality of electrodes. Each electrode of the plurality of electrodes can be attached to a spine of the plurality of spines. The medical probe can further include an irrigation hub attached to the tubular shaft and configured to receive and support the plurality of spines. The irrigation hub can comprise a cylindrical member extending along the longitudinal axis and comprising a proximal end having a first outer diameter and a recess extending inwardly along the longitudinal axis forming an interior portion and a distal end having a second outer diameter. The second outer diameter can be less than the first outer diameter. The cylindrical member can include an irrigation inlet chamber disposed proximate the interior portion and configured to receive fluid from an irrigation supply line separate from the tubular shaft to prevent fluid immersion into the tubular shaft, a plurality of irrigation openings disposed generally transverse to the longitudinal axis from a distal portion of the irrigation inlet chamber, and a flow diverter extending into the distal portion of the irrigation inlet chamber to block fluid flow and redirect fluid out of the plurality of irrigation openings in a direction generally transverse relative to the longitudinal axis.

Additional features, functionalities, and applications of the disclosed technology are discussed in more detail herein.

### BRIEF DESCRIPTION OF THE DRAWINGS

FIG. 1 is a schematic pictorial illustration of a medical system including a medical probe with electrodes, in accordance with the disclosed technology;
FIG. 2 is a schematic pictorial illustration showing a perspective view of a medical probe with electrodes in an expanded form, in accordance with the disclosed technology;
FIG. 3 is a schematic pictorial illustration showing an exploded view of a medical probe, in accordance with the disclosed technology;
FIG. 4A is a schematic pictorial illustration showing a top perspective view of an irrigation hub, in accordance with the disclosed technology;
FIG. 4B is a schematic pictorial illustration showing a bottom perspective view of an irrigation hub, in accordance with the disclosed technology;
FIG. 5A is a schematic pictorial illustration showing a side view of an irrigation hub, in accordance with the disclosed technology;
FIG. 5B is a schematic pictorial illustration showing a top view of an irrigation hub, in accordance with the disclosed technology;
FIG. 5C is a schematic pictorial illustration showing a bottom view of an irrigation hub, in accordance with the disclosed technology;
FIG. 6 is a schematic pictorial illustration showing a sectional view of an irrigation hub, in accordance with the disclosed technology;
FIG. 7 is a schematic pictorial illustration showing flow of a fluid through an irrigation hub, in accordance with the disclosed technology;
FIG. 8A is a schematic pictorial illustration showing a perspective view of another example medical probe with electrodes in an expanded form, in accordance with another example of the disclosed technology; and
FIG. 8B is a schematic pictorial illustration showing a perspective view of the medical probe of 8A showing the spines, in accordance with the disclosed technology.

### DETAILED DESCRIPTION

The following detailed description should be read with reference to the drawings, in which like elements in different drawings are identically numbered. The drawings, which are not necessarily to scale, depict selected examples and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%. In addition, as used herein, the terms "patient," "host," "user," and "subject" refer to any human or animal subject and are not intended to limit the systems or methods to human use, although use of the subject invention in a human patient represents a preferred embodiment. As well, the term "proximal" indicates a location closer to the operator or physician whereas "distal" indicates a location further away to the operator or physician.

As discussed herein, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "physician" can include a doctor, surgeon, technician, scientist, operator, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as reversable or irreversible electroporation (IRE), referred throughout this disclosure interchangeably as pulsed electric field (PEF) and pulsed field ablation (PFA), or thermal energy such as radiofrequency (RF) ablation or cryoablation. Ablating or ablation as it relates to the devices and corresponding systems of this disclosure is used throughout this disclosure in reference to thermal or non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

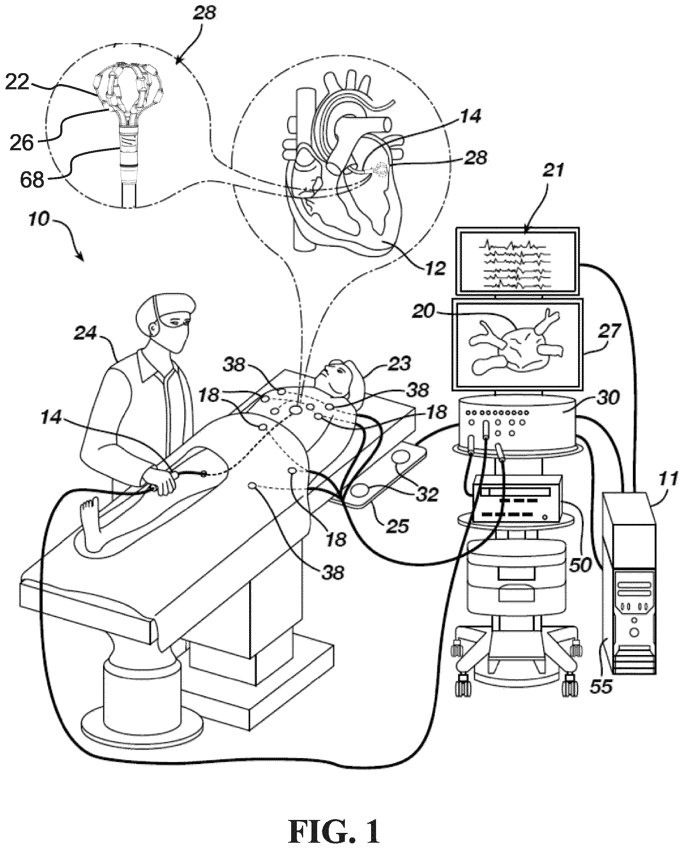

FIG. 1 shows an example catheter-based electrophysiology mapping and ablation system 10. System 10 includes multiple catheters, which are percutaneously inserted by physician 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example catheter 14 that is configured for sensing IEGM is illustrated herein. Physician 24 brings a distal tip of catheter 14 (i.e., a basket catheter 28 in this case) into contact with the heart wall for sensing a target site in heart 12. For ablation, physician 24 would similarly bring a distal end of an ablation catheter to a target site for ablating.

Catheter 14 is an exemplary catheter that includes one and preferably multiple electrodes 26 optionally distributed over a plurality of spines 22 at basket catheter 28 and configured to sense the IEGM signals. Catheter 14 may additionally include a position sensor 29 embedded in or near basket catheter 28 for tracking position and orientation of basket catheter 28. Optionally and preferably, position sensor 29 is a magnetic based position sensor including three magnetic coils for sensing three-dimensional (3D) position and orientation.

A combination magnetic based position sensor and force sensor 68 may be operated together with a location pad 25 including a plurality of magnetic coils 32 configured to generate magnetic fields in a predefined working volume. Real time position of basket catheter 28 of catheter 14 may be tracked based on magnetic fields generated with location pad 25 and sensed by magnetic based position sensor 29. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, each of which are incorporated herein by reference and attached in the Appendix hereto.

System 10 includes one or more electrode patches 38 positioned for skin contact on patient 23 to establish location reference for location pad 25 as well as impedance-based tracking of electrodes 26. For impedance-based tracking, electrical current is directed toward electrodes 26 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182, each of which are incorporated herein by reference and attached in the Appendix hereto.

A recorder 11 displays electrograms 21 captured with body surface ECG electrodes 18 and intracardiac electrograms (IEGM) captured with electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (5) displaying on display device 27 sites of interest such as places where ablation energy has been applied. One commercial product embodying elements of the system 10 is available as the CARTO^{™} 3 System, available from Biosense Webster, Inc., 31 Technology Drive, Suite 200, Irvine, CA 92618, USA.

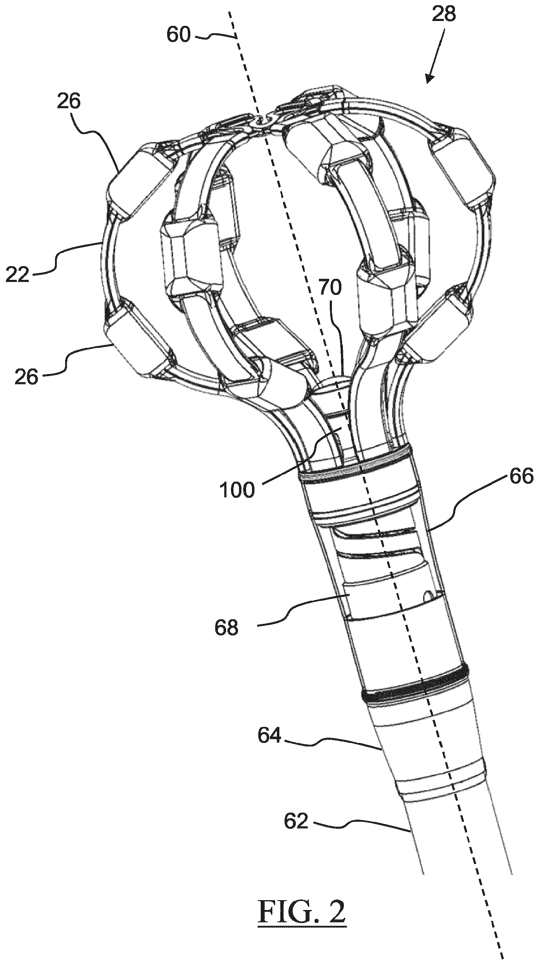

FIG. 2 is a schematic pictorial illustration showing a perspective view of a basket catheter 28 with electrodes 26 attached to spines 22 that are shown in an expanded form, such as by being advanced out of a tubular shaft lumen at a distal end of an insertion tube, in accordance with an embodiment of the present invention. As shown in FIG. 2, the basket catheter 28 includes a plurality of flexible spines 22 that are formed at the end of a tubular shaft 62 and are connected at both ends. The spines 22 can be configured to bow radially outward from a longitudinal axis 60 passing through the basket catheter 28. During a medical procedure, physician 24 can deploy basket catheter 28 by extending tubular shaft 62 from an insertion tube causing the basket catheter 28 to exit the insertion tube and transition to the expanded form. Spines 22 may have elliptical (e.g., circular) or rectangular (that may appear to be flat) cross-sections, and include a flexible, resilient material (e.g., a shape-memory alloy such as nickel-titanium, also known as Nitinol) forming a strut.

The physician 24 can bring the basket catheter 28 into contact with tissue to perform the ablation procedure. As ablative energy is output by the electrodes 26, the electrodes 26 and nearby tissue may begin to be heated. To help dissipate the heat generated by the electrodes 26, the disclosed technology can include an irrigation hub 100 that can be configured to deliver a fluid proximate the electrodes 26 to cool the electrodes 26 and prevent thrombosis, as will be described in greater detail herein.

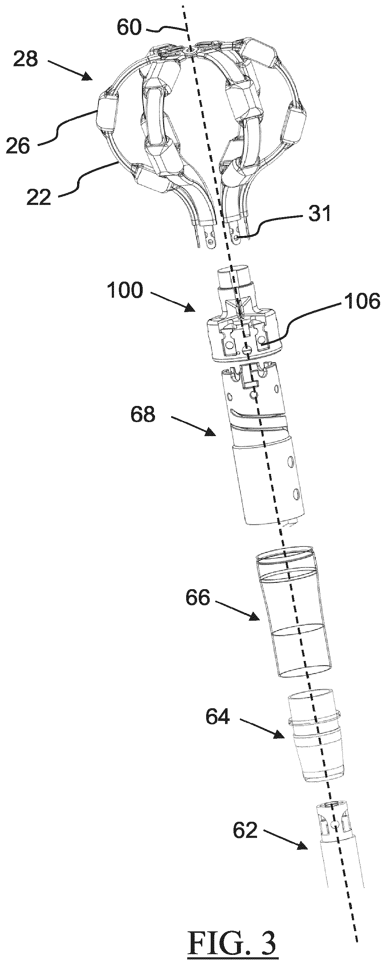

As shown in FIGs. 2 and 3, the basket catheter 28 can be attached to the tubular shaft 62 by a coupler 64 and a sleeve 66 positioned between the tubular shaft 62 and the basket catheter 28. Additionally, a combination magnetic based position sensor and force sensor 68 can be positioned between the tubular shaft 62 and the basket catheter 28 and be configured to detect a force applied to the basket catheter 28. The combination sensor 68 can be disposed at least partially within the sleeve 66.

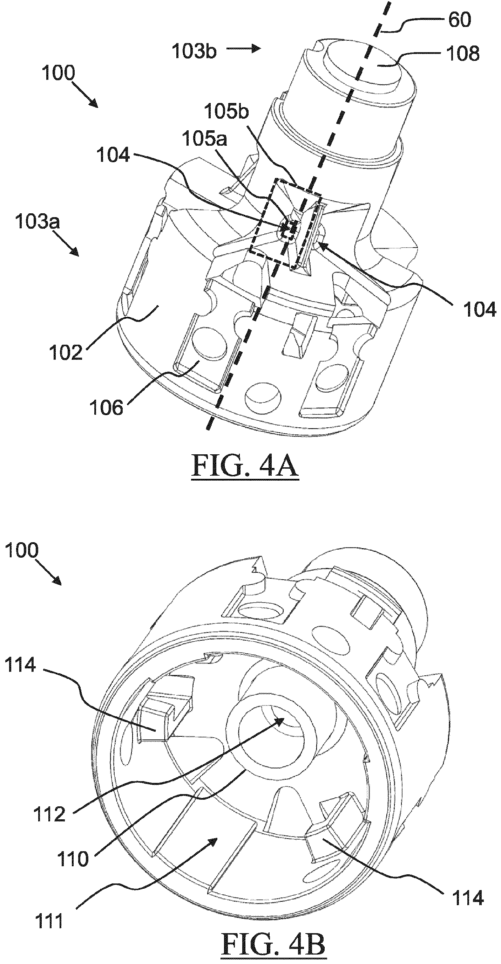

FIG. 4A is a schematic pictorial illustration showing a top perspective view of an irrigation hub 100 while FIG. 4B is a schematic pictorial illustration showing a bottom perspective view of an irrigation hub 100, in accordance with the disclosed technology. As previously mentioned, and as will be described in greater detail herein, the irrigation hub 100 can be configured to deliver a fluid to the electrodes 26 of the basket catheter 28. As shown in FIG. 4A, the irrigation hub 100 can include a cylindrical member 102 comprising a proximal end 103a and a distal end 103b. As shown, the proximal end 103a can have an outer diameter that is greater than an outer diameter of the distal end 103b.

The irrigation hub 100 can include a plurality of irrigation openings 104 that can be configured to permit fluid to flow therethrough and to help direct the fluid outwardly from the irrigation hub 100. The irrigation openings 104 can be dispersed radially around the distal end 103b and be generally transverse to the longitudinal axis 60. The irrigation openings 104 can each form an aperture having an inlet area 105a that is smaller than an outlet area 105b such that the fluid is permitted to disperse outwardly when directed out of the irrigation openings 104. In other words, as fluid flows through the irrigation hub 100 and out of the irrigation openings 104, the inlet area 105a through which the fluid first flows through the irrigation openings will be smaller than the outlet area 105b through which the fluid flows just prior to leaving the irrigation hub 100. In this way, the irrigation hub 100 can help to guide or direct the irrigation fluid toward the electrodes 26 or otherwise outwardly from the irrigation hub 100.

The irrigation hub 100 can further include a plurality of relief lands 106 that can be configured to receive and help retain the spines 22. As shown in FIG. 3, the spines 22 can each include a connection end 31 that can be configured to be at least partially inserted into the relief lands 106 such that the spines 22 can be secured in place with assembled with the irrigation hub 100.

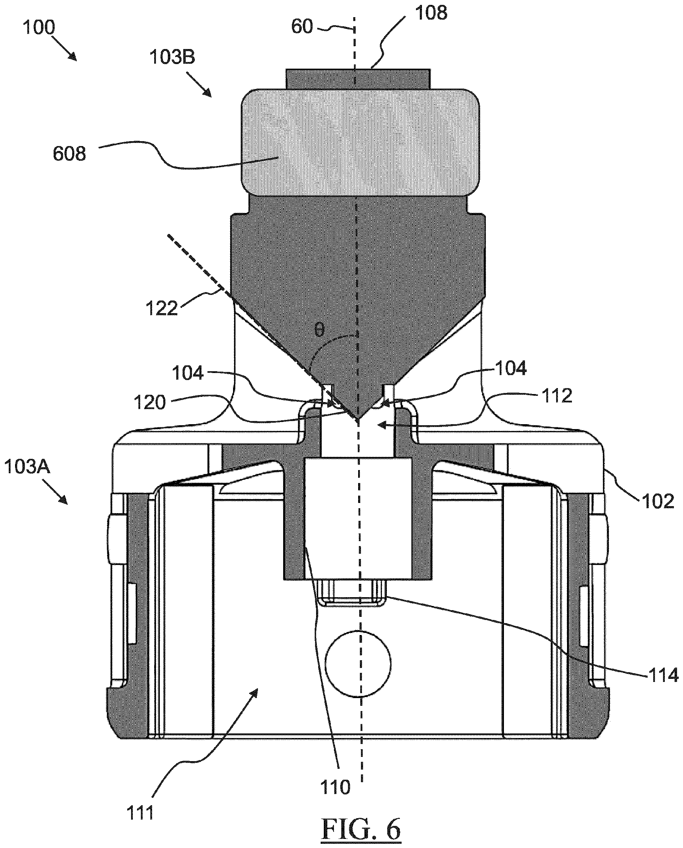

The irrigation hub 100 can further include a sensor mount 108 that can be disposed at the distal end 103b of the cylindrical member 102. The sensor mount 108 can be configured to receive and support one or more sensors 70, 608 (see FIGs. 2 and 6) of the medical probe. In some examples, the sensors can be a reference electrode configured to detect far field signals that can be used in processing and filtering signals detected by the electrodes 26 when, for example, the electrodes 26 are used for mapping of electrical signals dispersed through a tissue. In other examples, the sensors can be or include one or more magnetic position sensors that can be used to detect magnetic fields output by one or more magnetic field generators to determine a position and/or orientation of the basket catheter 28.

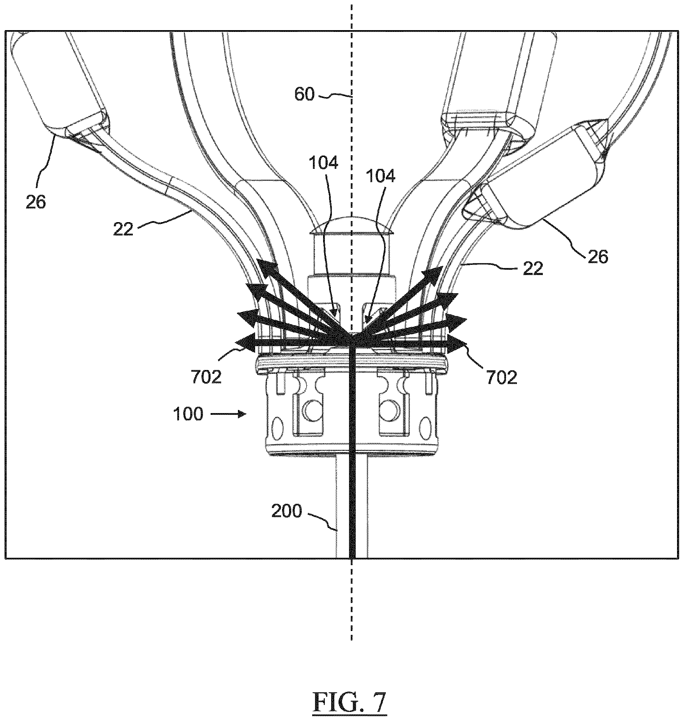

As shown in FIG. 4B, the proximal end 103a of the cylindrical member 102 can include a recess extending inwardly along the longitudinal axis and forming an interior portion 111. The irrigation hub 100 can further include an irrigation coupler 110 that can be configured to receive, or otherwise be connected to, an irrigation supply tube 200 (as shown in FIG. 7). The cylindrical member 102 can further include an irrigation inlet chamber 112 that can be disposed distal the irrigation coupler 110 and can be configured to receive fluid from the irrigation supply tube 200. The irrigation supply tube 200 can fluidly separate the fluid from the interior portion 111, the combination sensor 68, the tubular shaft 62 and other components of the medical probe. In other words, the fluid can be delivered to the irrigation inlet chamber 112 via the irrigation supply tube 200 without the fluid coming into contact with other interior components of the medical probe. The irrigation inlet chamber 112 can be sized to receive a sufficient amount of fluid from the irrigation supply tube 200 such that the flow of fluid is generally not impeded. In some examples, the irrigation inlet chamber 112 can have an inner diameter that is equal to the inner diameter of the irrigation supply tube 200. The irrigation inlet chamber 112 can be fluidly connected to the plurality of irrigation openings 104 such that the fluid can flow through the irrigation inlet chamber 112 and be directed out of the plurality of irrigation openings 104.

The irrigation hub 100 can further include a plurality of attachment mechanisms 114 that can be configured for attaching the irrigation hub 100 to the combination sensor 68 and/or the tubular shaft 62. The attachment mechanisms 114 can be, for example and not limited to, bayonet mounts, snap connectors, a threaded fitting, or other suitable types of attachment mechanisms 114 for the particular application.

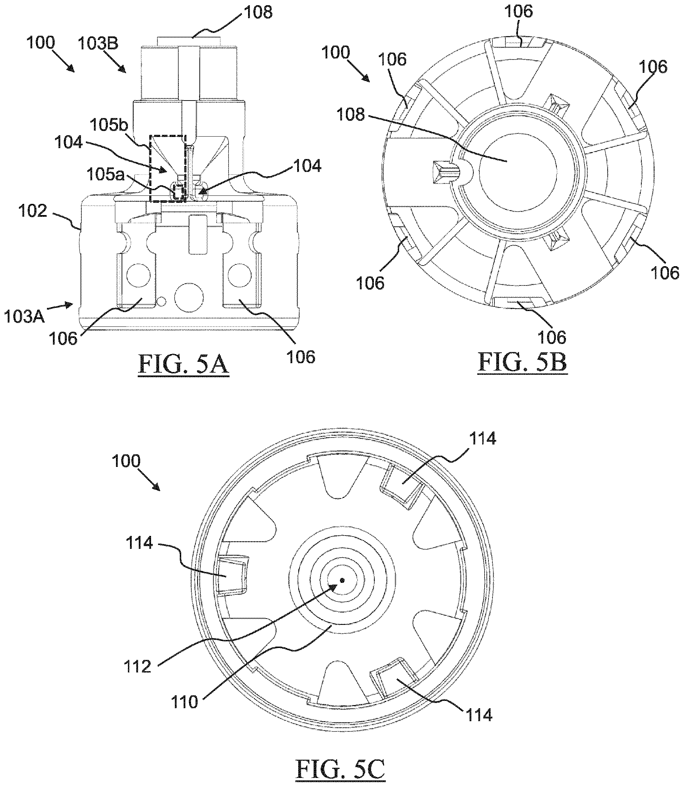

FIGs. 5A-5C illustrate various views of the irrigation hub 100. In particular, FIG. 5A illustrates a side view, FIG. 5B illustrates a top view, and FIG. 5C illustrates a bottom view of the irrigation hub 100, in accordance with the disclosed technology. Each of the reference numerals shown in FIGs. 5A-5C correspond to the various components and/or features described herein.

FIG. 6 shows a sectional view of the irrigation hub 100, in accordance with the disclosed technology. As shown in FIG. 6, the irrigation hub 100 can include a flow diverter 120 disposed at a distal end of the irrigation inlet chamber 112 and extends inwardly into the irrigation inlet chamber 112. The flow diverter 120, in some examples, can be a conical member that has an outer surface that extends at an angle θ away from the longitudinal axis. The angle θ can be a predetermined angle sufficient to redirect the fluid received from the irrigation supply tube 200 out the plurality of irrigation openings 104 such that the fluid is directed generally transverse to the longitudinal axis 60. In some examples, the angle θ can direct the fluid toward the electrodes 26. As non-limiting examples, the angle θ can be approximately 15°, 20°, 25°, 30°, 35°, 40°, 45°, 60°, 75°, 85°, or any other suitable angle for the particular application. Although described as a conical member, the flow diverter can comprise other shapes having generally planar sides, generally curves sides, or other configurations in which the fluid can be directed by the flow diverter 120 outwardly through the plurality of irrigation openings 104.

As will be appreciated, the irrigation openings 104 can extend outwardly from the irrigation inlet chamber 112 through the irrigation hub 100. As described previously, the irrigation openings 104 can include an inlet area 105a that is smaller than an outlet area 105b. the inlet area 105a can be near the irrigation inlet chamber 112 and the outlet area 105b can be disposed a distance away from the irrigation inlet chamber 112. A surface 122 of the irrigation openings 104 can extend between the inlet are 105a and the outlet are 105b. The surface 122 can be configured such that the surface is disposed at the angle θ or an angle that is substantially similar to the angle θ such that the fluid can be directed outwardly through the irrigation openings 104 without generating significant turbulence.

FIG. 6 further shows a sensor 608 attached to the sensor mount 108. As described *supra,* the sensor can be a magnetic position sensor, a reference electrode, or any other sensor for the particular configuration. Although the sensor 608 is shown as being disposed around or through the sensor mount 108, the sensor 608 can also be disposed at the very distal end of the sensor mount 108 (as shown in FIG. 2, sensor 70) or a first sensor 70 can be disposed at the very distal end of sensor mount 108 and a second sensor 608 can be disposed around or through the sensor mount 108. The first sensor 70 and the second sensor 608 can be the same type or different types of sensors. In other examples, the sensor mount 108 can be configured to receive and support multiple sensors (e.g., a first sensor disposed around the sensor mount 108 and a second sensor disposed at the distal end of the sensor mount 108).

FIG. 7 illustrates a flow path 702 of a fluid through an irrigation hub 100, in accordance with an embodiment of the present invention. As shown in FIG. 7, the irrigation fluid can have a flow path 702 that extends through the irrigation supply tube 200 and is redirected by the irrigation hub 100 outwardly. In some examples, the irrigation hub 100 can redirect the fluid generally transverse to the longitudinal axis 60. In other examples, the irrigation hub 100 can redirect the fluid at other angles as described herein to obtain the desired cooling effect at the electrodes.

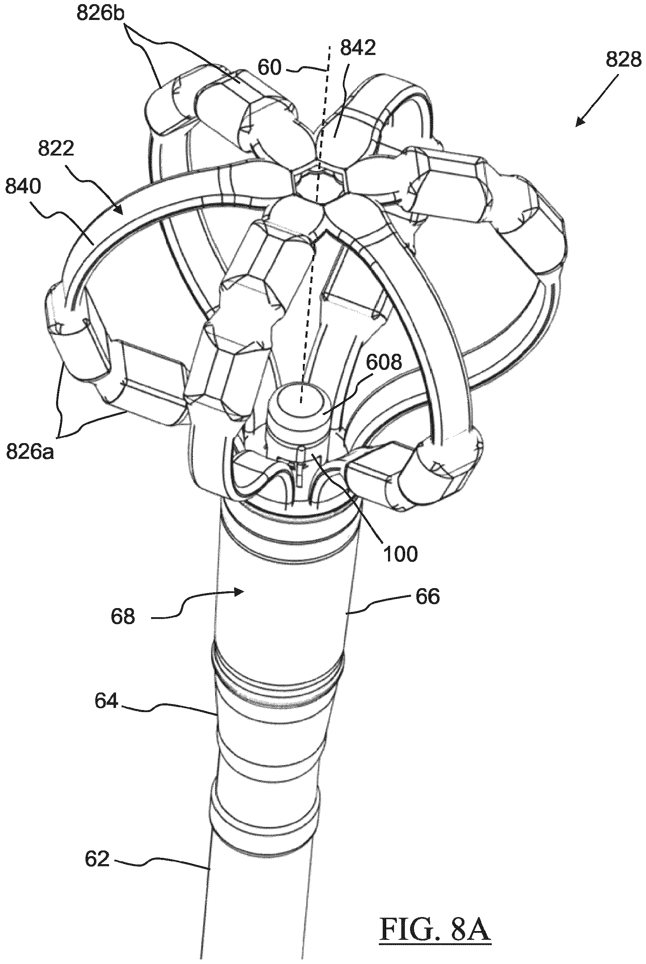

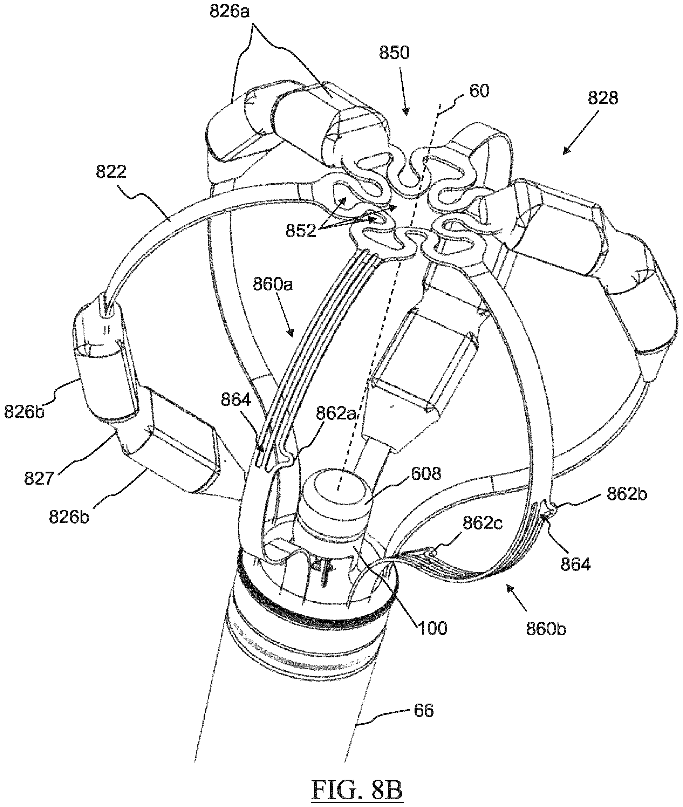

FIGs. 8A and 8B illustrate another example basket catheter 828 having a plurality of electrodes 826 disposed on spines 822 and an irrigation hub 100 having a sensor 608 mounted thereon. As shown in FIG. 8A, the electrodes 826 can be disposed in alternating groupings of distal electrodes 826a and proximal electrodes 826b on adjacent spines 822. For example, and as shown in FIGs. 8A and 8B, two electrodes 826a, 826b can be disposed on the spines 822 close to each other with no additional electrodes 826 disposed on the same spine 822. On a first spine 822, the two electrodes 826b can be disposed together near the proximal end of the spine 822 while on a second, adjacent spine 822 two electrodes 826a can be disposed together near the distal end of the adjacent spines 822. In this way, the electrodes 826a, 826b can be offset around the circumference of the basket catheter 828 such that the basket catheter 826 is better able to collapse when retracted into a sheath. When the basket catheter 828 is collapsed, the distal electrodes 826a are positioned entirely in a distal direction from the proximal electrodes 826b with a gap along the longitudinal axis 60 between the proximal electrodes 826b and the distal electrode 826a.

With the configuration of electrodes 826a, 826b disposed on the spines 822 as shown in FIGs. 8A and 8B, the system 10 can be configured to output bipolar high-voltage DC pulses as may be used to effect irreversible electroporation (IRE) between the two adjacent electrodes 826a, 826b on a given spine 822, electrically connect the two adjacent electrodes 826 on a given spine 822 and output bipolar high-voltage DC pulses between one or more electrodes 822 on another one of the spines 822 of the basket catheter 828, and/or output monopolar high-voltage DC pulses between one or more of the electrodes 828 and the one or more electrode patches 38 disposed on the patient's 23 skin. The two electrodes 826 on a given spine 822 can include an insulative material 827 disposed between the two electrodes 826a 826b, thereby electrically isolating the two electrodes 826a 826b from each other.

As shown in FIG. 8A, the spines 822 can be covered with an insulative liner 840 that can be disposed between the electrodes 826 and the spines 822. The insulative liner 840 can electrically isolate the electrodes 826 from the spines 822 to prevent arcing or shorting to the spines 822. The insulative liner 840 can extend from the irrigation hub 100 to a distal end of the basket catheter 828. Furthermore, the insulative liner 840 can include flared ends 842 that can extend over at least a portion of the central spine intersection 850. In this way, the insulative liner 840 can have an atraumatic tip to prevent injury to tissue.

FIG. 8B is an illustration of the basket catheter 828 with the insulative liners 840, a pair of distal electrodes 826a, and a pair of proximal electrodes 826b, and other spine elements removed, for the sake of illustration, so that the frame of the basket catheter 828 is visible. As shown in FIG. 8B, the spines 822 can extend from the irrigation hub 100 and be joined together at a central spine intersection 850. The central spine intersection 850 can include one or more cutouts 852 that can allow for bending of the spines 822. The spines 822 can further include an electrode retention region 860a, 860b that is configured to prevent an electrode 826a, 826b from sliding proximally or distally along the spine 822. As shown in FIG. 8B, a first spine 822 can have distal spine retention region 860a and an adjacent spine can have a proximal spine retention region 860b. In this way, the spine retention regions 860a, 860b can be alternating between a proximal position and a distal position along the spines 822. That is, a first spine 822 can have an electrode retention region 860b disposed near a proximal end of the spine 822 and an adjacent spine 822a can have an electrode retention region 860 disposed near a distal end of the spine 822.

Each electrode retention region 860 can include one or more cutouts 864 that can permit the spine 822 to be bent or pinched inwardly. Each electrode retention region 860 can further include one or more retention members 862 that protrude outwardly and can be configured to prevent the electrode 826 from sliding proximally or distally along the spine 822. During manufacture, proximal ends of the frame of the basket catheter 828 are inserted into lumens of the electrodes 826a, 826b, and the electrodes 826a, 826b are slid distally along the spines 822 to their respective final position. The cutouts 864 permit the electrodes 826a, 826b to slide over a retention members 862a-c. Because of the one or more cutouts 864 in the spines 822, the retention members 862a-c can be configured to move inwardly when the spine 822 is pinched inwardly to permit an electrode 826a, 826b to slide over the retention member 862a-c. Once the electrode 826a, 826b is slid past the retention member 862, the retention member 862 can resiliently bend back to its previous position, thereby preventing the electrode 826a, 826b from sliding proximally or distally along the spine 822.

The proximal electrode retention region 860b includes a proximal retention member 862c and a distal retention member 862b. The proximal electrode retention region 860b need not be configured to permit the proximal electrodes 826b to pass over the distal retention member 862b. The distal electrode retention region 860a utilizes the central spine intersection 850 to prevent the distal electrodes 826a from moving distally once the distal electrodes 826a are in their respective final position.

Although the basket catheter 828 is shown as having two electrodes 826 disposed near each other on a given spine 822 and having alternating groupings of electrodes 826 on adjacent spines 822, the disclosed technology can include other configurations of electrodes 826 and spines 822 not shown. For example, the disclosed technology can include groupings of three or more electrodes 826 and/or multiple groupings of electrodes 826 disposed on spines 822. Thus, the disclosed technology is not limited to the particular configuration of electrodes 826 and spines 822 shown and described herein.

The disclosed technology described herein can be further understood according to the following clauses:
Clause 1: An irrigation hub for an ablation catheter, the irrigation hub comprising: a cylindrical member extending along a longitudinal axis, the cylindrical member comprising: a proximal end having a first outer diameter and a recess extending inwardly along the longitudinal axis forming an interior portion; a distal end having a second outer diameter, the second outer diameter being less than the first outer diameter; an irrigation inlet chamber disposed proximate the interior portion and configured to receive fluid from an irrigation supply; a plurality of irrigation openings disposed generally transverse to the longitudinal axis from a distal portion of the irrigation inlet chamber; and a flow diverter extending into the distal portion of the irrigation inlet chamber to block fluid flow and redirect fluid flow out of the plurality of irrigation openings in a direction generally transverse relative to the longitudinal axis.
Clause 2: The irrigation hub of Clause 1, wherein the plurality of irrigation openings are disposed radially around the cylindrical member and are configured to direct the fluid toward electrodes of a basket catheter.
Clause 3: The irrigation hub of Clauses 1 or 2, wherein each irrigation opening of the plurality of irrigation openings comprises an aperture having an outlet area greater than an inlet area.
Clause 4: The irrigation hub of any of Clauses 1-3, wherein the flow diverter comprises a conical member extending proximally along the longitudinal axis into the irrigation inlet chamber.
Clause 5: The irrigation hub of any of Clauses 1-4, wherein at least a portion of each irrigation opening extends outwardly at an angle.
Clause 6: The irrigation hub of Clause 5, wherein the angle of each irrigation opening relative to the longitudinal axis is approximately equal to an angle formed by an outer surface of the conical member relative to the longitudinal axis.
Clause 7: The irrigation hub of any of Clauses 1-6, wherein the proximal end comprises one or more attachment mechanisms configured to releasably attach the proximal end to a catheter shaft.
Clause 8: The irrigation hub of any of Clauses 1-6, wherein the proximal end comprises one or more attachment mechanisms configured to releasably attach the proximal end to a force sensor.
Clause 9: The irrigation hub of Clauses 7 or 8, wherein the one or more attachment mechanisms comprises one or more bayonet mounts.
Clause 10: The irrigation hub of any of the preceding Clauses, wherein the proximal end further comprises a plurality of relief lands disposed radially around an outer surface of the proximal end, each relief land of the plurality of relief lands configured to receive a spine of a basket catheter.
Clause 11: The irrigation hub of any of the preceding Clauses, wherein the distal end further comprises a sensor mount configured to receive and support a sensor.
Clause 12: The irrigation hub of Clause 11, wherein the sensor comprises a reference electrode.
Clause 13: The irrigation hub of Clause 11, wherein the sensor comprises a position sensor.
Clause 14: A medical probe, comprising: a tubular shaft extending along a longitudinal axis of the medical probe; a plurality of spines configured to bow radially outward from the longitudinal axis; a plurality of electrodes, each electrode of the plurality of electrodes attached to a spine of the plurality of spines; and an irrigation hub attached to the tubular shaft and configured to receive and support the plurality of spines, the irrigation hub comprising a cylindrical member extending along the longitudinal axis and comprising: a proximal end having a first outer diameter and a recess extending inwardly along the longitudinal axis forming an interior portion; a distal end having a second outer diameter, the second outer diameter being less than the first outer diameter; an irrigation inlet chamber disposed proximate the interior portion and configured to receive fluid from an irrigation supply line separate from the tubular shaft to prevent fluid immersion into the tubular shaft; a plurality of irrigation openings disposed generally transverse to the longitudinal axis from a distal portion of the irrigation inlet chamber; and a flow diverter extending into the distal portion of the irrigation inlet chamber to block fluid flow and redirect fluid out of the plurality of irrigation openings in a direction generally transverse relative to the longitudinal axis.
Clause 15: The medical probe of Clause 14, wherein the plurality of spines are configured to transition between an expanded state and a collapsed state.
Clause 16: The medical probe of Clauses 14 or 15, wherein the flow diverter is disposed at least partially in the irrigation inlet chamber.
Clause 17: The medical probe of any of Clauses 14-16, wherein the plurality of irrigation openings are disposed radially around the cylindrical member and are configured to direct the fluid toward the plurality of electrodes.
Clause 18: The medical probe of Clause 17, each electrode of the plurality of electrodes having a tissue-facing surface and an inwardly-facing surface, the plurality of irrigation openings configured to direct the fluid toward the inwardly-facing surface of each electrode of the plurality of electrodes.
Clause 19: The medical probe of any of Clauses 14-18, wherein each irrigation opening of the plurality of irrigation openings comprises an aperture having an outlet area greater than an inlet area.
Clause 20: The medical probe of any of Clauses 14-19, wherein the flow diverter comprises a conical member extending proximally along the longitudinal axis into the irrigation inlet chamber.
Clause 21: The medical probe of any of Clauses 14-19, wherein at least a portion of each irrigation opening extends outwardly at an angle.
Clause 22: The medical probe of Clause 21, wherein the angle of each irrigation opening relative to the longitudinal axis is approximately equal to an angle formed by an outer surface of the conical member relative to the longitudinal axis.
Clause 23: The medical probe of any of Clauses 14-22, wherein the proximal end comprises one or more attachment mechanisms configured to releasably attach the proximal end to the tubular shaft.
Clause 24: The medical probe of any of Clauses 14-22 further comprising a force sensor disposed between the irrigation hub and the tubular shaft.
Clause 25: The medical probe of Clause 24, wherein the proximal end comprises one or more attachment mechanisms configured to releasably attach the proximal end to the force sensor.
Clause 26: The medical probe of Clauses 23 or 25, wherein the one or more attachment mechanisms comprises one or more bayonet mounts.
Clause 27: The medical probe of any of Clauses 14-26, wherein the proximal end further comprises a plurality of relief lands disposed radially around an outer surface of the proximal end, each relief land of the plurality of relief lands configured to receive a respective spine of the plurality of spines.
Clause 28: The medical probe of any of Clauses 14-27, wherein the distal end further comprises a sensor mount configured to receive and support a sensor.
Clause 29: The medical probe of Clause 28, wherein the sensor comprises a reference electrode.
Clause 30: The medical probe of Clause 28, wherein the sensor comprises a position sensor.
Clause 31: A medical probe, comprising: a tubular shaft extending along a longitudinal axis of the medical probe; a plurality of spines configured to bow radially outward from the longitudinal axis, each spine of the plurality of spines comprising a retention member; a plurality of electrodes, each electrode of the plurality of electrodes attached to a spine of the plurality of spines and prevented from sliding proximally or distally along the spine by the retention member, the plurality of electrodes being disposed on the plurality of spines in groupings, the groupings being disposed in alternating proximal and distal positions along adjacent spines; and an irrigation hub attached to the tubular shaft and configured to receive and support the plurality of spines, the irrigation hub comprising a cylindrical member extending along the longitudinal axis and comprising: a plurality of irrigation openings disposed generally transverse to the longitudinal axis from a distal portion of an irrigation inlet chamber of the irrigation hub; and a flow diverter configured to block fluid flow and redirect fluid out of the plurality of irrigation openings in a direction generally transverse relative to the longitudinal axis.

The embodiments described above are cited by way of example, and the present invention is not limited by what has been particularly shown and described hereinabove. Rather, the scope of the invention includes both combinations and sub combinations of the various features described hereinabove, as well as variations and modifications thereof which would occur to persons skilled in the art upon reading the foregoing description and which are not disclosed in the prior art.

## Claims

1. An irrigation hub for an ablation catheter, the irrigation hub comprising:
a cylindrical member extending along a longitudinal axis, the cylindrical member comprising:
a proximal end having a first outer diameter and a recess extending inwardly along the longitudinal axis forming an interior portion;
a distal end having a second outer diameter, the second outer diameter being less than the first outer diameter;
an irrigation inlet chamber disposed proximate the interior portion and configured to receive fluid from an irrigation supply;
a plurality of irrigation openings disposed generally transverse to the longitudinal axis from a distal portion of the irrigation inlet chamber; and
a flow diverter extending into the distal portion of the irrigation inlet chamber to block fluid flow and redirect fluid flow out of the plurality of irrigation openings in a direction generally transverse relative to the longitudinal axis.

2. The irrigation hub of claim 1, wherein the plurality of irrigation openings are disposed radially around the cylindrical member and are configured to direct the fluid toward electrodes of a basket catheter.

3. The irrigation hub of claim 1 or claim 2, wherein each irrigation opening of the plurality of irrigation openings comprises an aperture having an outlet area greater than an inlet area.

4. The irrigation hub of claim 1, wherein the flow diverter comprises a conical member extending proximally along the longitudinal axis into the irrigation inlet chamber, optionally wherein at least a portion of each irrigation opening extends outwardly at an angle, further optionally wherein the angle of each irrigation opening relative to the longitudinal axis is approximately equal to an angle formed by an outer surface of the conical member relative to the longitudinal axis.

5. The irrigation hub of any preceding claim, wherein the proximal end comprises one or more attachment mechanisms configured to releasably attach the proximal end to a catheter shaft.

6. The irrigation hub of any preceding claim, wherein the proximal end comprises one or more attachment mechanisms configured to releasably attach the proximal end to a force sensor, optionally wherein the one or more attachment mechanisms comprises one or more bayonet mounts.

7. The irrigation hub of any preceding claim, wherein the proximal end further comprises a plurality of relief lands disposed radially around an outer surface of the proximal end, each relief land of the plurality of relief lands configured to receive a spine of a basket catheter.

8. The irrigation hub of any preceding claim, wherein the distal end further comprises a sensor mount configured to receive and support a sensor.

9. The irrigation hub of claim 8, wherein the sensor comprises a reference electrode, or a position sensor.

10. A medical probe, comprising:
a tubular shaft extending along a longitudinal axis of the medical probe;
a plurality of spines configured to bow radially outward from the longitudinal axis;
a plurality of electrodes, each electrode of the plurality of electrodes attached to a spine of the plurality of spines; and
an irrigation hub attached to the tubular shaft and configured to receive and support the plurality of spines, the irrigation hub comprising a cylindrical member extending along the longitudinal axis and comprising:
a proximal end having a first outer diameter and a recess extending inwardly along the longitudinal axis forming an interior portion;
a distal end having a second outer diameter, the second outer diameter being less than the first outer diameter;
an irrigation inlet chamber disposed proximate the interior portion and configured to receive fluid from an irrigation supply line separate from the tubular shaft to prevent fluid immersion into the tubular shaft;
a plurality of irrigation openings disposed generally transverse to the longitudinal axis from a distal portion of the irrigation inlet chamber; and
a flow diverter extending into the distal portion of the irrigation inlet chamber to block fluid flow and redirect fluid out of the plurality of irrigation openings in a direction generally transverse relative to the longitudinal axis.

11. The medical probe of claim 10, wherein the plurality of spines are configured to transition between an expanded state and a collapsed state.

12. The medical probe of claim 10 or claim 11, wherein the plurality of irrigation openings are disposed radially around the cylindrical member and are configured to direct the fluid toward the plurality of electrodes.

13. The medical probe of claim 10, each electrode of the plurality of electrodes having a tissue-facing surface and an inwardly-facing surface, the plurality of irrigation openings configured to direct the fluid toward the inwardly-facing surface of each electrode of the plurality of electrodes.

14. The medical probe of any of claims 10 to 13 further comprising a force sensor disposed between the irrigation hub and the tubular shaft, optionally wherein the proximal end comprises one or more attachment mechanisms configured to releasably attach the proximal end to the force sensor.

15. A medical probe, comprising:
a tubular shaft extending along a longitudinal axis of the medical probe;
a plurality of spines configured to bow radially outward from the longitudinal axis, each spine of the plurality of spines comprising a retention member;
a plurality of electrodes, each electrode of the plurality of electrodes attached to a spine of the plurality of spines and prevented from sliding proximally or distally along the spine by the retention member, the plurality of electrodes being disposed on the plurality of spines in groupings, the groupings being disposed in alternating proximal and distal positions along adjacent spines; and
an irrigation hub attached to the tubular shaft and configured to receive and support the plurality of spines, the irrigation hub comprising a cylindrical member extending along the longitudinal axis and comprising:
a plurality of irrigation openings disposed generally transverse to the longitudinal axis from a distal portion of an irrigation inlet chamber of the irrigation hub; and
a flow diverter configured to block fluid flow and redirect fluid out of the plurality of irrigation openings in a direction generally transverse relative to the longitudinal axis.
